(19) Europäisches Patentamt
European Patent Office
Office européen des brevets

(11) EP 4 079 960 B1

(12) **EUROPEAN PATENT SPECIFICATION**

(45) Date of publication and mention of the grant of the patent:
**29.07.2026 Bulletin 2026/31**

(21) Application number: **20900909.1**

(22) Date of filing: **18.12.2020**

(51) International Patent Classification (IPC):
***D06L 1/12*** (2006.01) ***D06M 13/256*** (2006.01)
***D06L 1/14*** (2006.01) ***D06M 13/224*** (2006.01)

(52) Cooperative Patent Classification (CPC):
**D06L 1/14; D06L 1/12; D06M 13/2246; D06M 13/256;** D06M 2200/50

(86) International application number:
**PCT/JP2020/047449**

(87) International publication number:
**WO 2021/125326 (24.06.2021 Gazette 2021/25)**

(54) **SOFTENING BASE AGENT**

WEICHMACHENDES BASISMITTEL

AGENT DE BASE ADOUCISSANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2019 JP 2019229980**

(43) Date of publication of application:
**26.10.2022 Bulletin 2022/43**

(73) Proprietor: **Kao Corporation**
**Chuo-ku**
**Tokyo 103-8210 (JP)**

(72) Inventors:
- **IGARASHI, Takako**
  **Wakayama-shi, Wakayama 640-8580 (JP)**
- **TAMAGAWA, Ojiro**
  **Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

(56) References cited:
**EP-A1- 4 190 883** **JP-A- 2005 171 399**
**JP-A- 2012 172 032** **JP-A- 2017 110 328**
**JP-A- H0 827 662** **JP-A- H0 827 662**
**JP-A- H08 158 258** **JP-A- H08 325 952**
**US-A- 4 176 080** **US-A1- 2007 214 999**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Invention

The present invention relates to a method for treating fibers comprising treating the fibers with a softening base agent.

Background of the Invention

Textile products such as clothing or the like gradually become stiff and undesirable in textures as they are repeatedly worn or washed. They are softened with fabric softeners added in rinsing processes during washing or the like to improve this.

While most softening agent compositions commercially available at present contain cationic surfactants as active ingredients, softening agent compositions containing anionic surfactants have been conventionally studied. For example, JP-A 2005-171399 discloses a softening agent composition consisting of a neutralized or quaternized product of an amine compound and a sulfonate-type anionic surfactant, the composition imparting excellent softness to various types of textile products without impairing the water absorbency of hydrophilic textile products. Further, JP-A H8-158258 discloses an antibacterial softening agent composition comprising a cationic bactericidal agent such as benzalkonium chloride or the like, an α-olefin sulfonate salt and/or a dialkyl sulfosuccinate salt as essential components, the composition imparting excellent textures to various types of fibers and exhibiting an excellent antibacterial effect. In addition, US-B 5419842 discloses a fabric softening aqueous emulsion comprising a higher fatty acid ester of pentaerythritol, an oligomer of pentaerythritol, a lower alkylene oxide derivative of pentaerythritol, a lower alkylene oxide derivative of an oligomer of pentaerythritol or a mixture of any two or more thereof as a fabric softening compound, the emulsion further comprising an anionic emulsifier selected from the group consisting of diisotridecyl sulfosuccinate, diisodecyl sulfosuccinate and an alkali metal salt of a fatty acid, an ethoxylated alcohol emulsifier and an aqueous medium in amounts falling within their respective predetermined ranges, wherein the emulsion is essentially free of cationic emulsifiers and fabric softeners. This literature states that a predetermined compound such as a higher fatty acid ester of pentaerythritol or the like is the only fabric softening compound other than bentonite. US 4 176 080 A relates to detergent compositions for effective oily soil removal.
JP H08 27662 A relates to a fiber softening base material, particularly a surfactant.
JP 2012 172032 A relates to a cleaning composition for dishwashing.
US 2007/214999 A1 relates to salts of alkyl esters of sulfonated dicarboxylic acids and compositions containing the same.

Summary of the Invention

While anionic surfactants are preferable components from the viewpoint of formulation as softening agent compositions using them cause less discoloration, softening base agents using anionic surfactants are desired to have further improved softening effects.

Further, fiber treatment agent compositions such as softening agent compositions or the like are generally formulated in the form of dispersions including water and desired to be stable in such forms.

The present invention is defined in and by the appended claims. The present invention is directed towards a method for treating fibers comprising treating the fibers with a softening base agent. The softening base agent uses an anionic surfactant, the softening base agent has excellent dispersion stability in formulations and is superior to other anionic surfactants in fiber softening effect.

The softening base agent for treating fibers contains a compound represented by the following formula 1 (hereinafter referred to as compound 1):

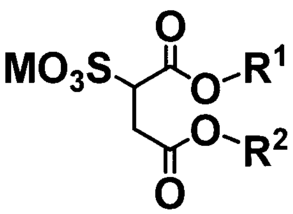

Formula 1

wherein each of $R^1$ and $R^2$ represents a hydrocarbon group with 6 or more and 17 or less carbons, and the total number of carbons in $R^1$ and $R^2$ is 18 or more and 30 or less; and M is a cation, excluding a hydrogen ion.

At least one of $R^1$ and $R^2$ in the formula 1 is a hydrocarbon group having a branched structure or an unsaturated

bond;

the hydrocarbon groups of $R^1$ and $R^2$ are different; and
the hydrocarbon group having a branched structure has a branched chain in the second position, and the branched chain is a hydrocarbon group with 2 or more carbons, wherein the second position is defined as a carbon of $R^1$ or $R^2$ bonded to the carbon in a first position, wherein the first position is defined as a carbon of $R^1$ or $R^2$ bonded to O of -O-$R^1$ or -O-$R^2$ in formula 1.

The present invention relates to a method for treating fibers including, treating the fibers with the softening base agent as outlined in appended claim 1, wherein the compound represented by the formula 1 is used in an amount of 0.01% o.w.f. or more and 5% o.w.f. or less relative to the fibers.
According to the present invention, provided is a softening base agent using an anionic surfactant, the softening base agent having excellent dispersion stability in formulations and being superior to other anionic surfactants in fiber softening effect.

Embodiments of the Invention

[Softening base agent]

The softening base agent means an agent (active ingredient) that develops softness to fibers.
The softening base agent contains compound 1 represented by the formula 1. The softening base agent may be composed of compound 1. Further, the softening base agent may contain one or more types of compound 1 or may be composed of one or more types of compound 1.
Compound 1 is a sulfosuccinate salt with two hydrocarbon groups having specific structures, and is excellent in dispersion stability in aqueous systems and capable of imparting excellent softness to fibers. The reason why compound 1 exhibits such effects is not clear, but it is considered as follows.
Generally, anionic surfactants of the sulfonate-type or the like having long alkyl chains are high in Krafft point and likely to be flocculated and separated in water. Uniform treatment of fibers with flocculating dispersions is difficult even if carrying out bathing treatment against them, so that performance is less likely to be developed. On the other hand, in the present invention, specific structures and specific hydrocarbon groups (for example, carbon numbers of alkyl groups and structures of alkyl groups) of compound 1 represented by the formula 1 optimize hydrophilicity/hydrophobicity, flocculating properties and curvature, and enable the preparation of stable water dispersions. It is inferred that uniform and efficient treatment of fibers can be carried out in such a stable dispersion state, resulting in the development of excellent fiber modifying effects. Note that the present invention is not restrained by this mechanism.
In the formula 1, $R^1$ and $R^2$ may be the same or different, and each of them is a hydrocarbon group with 6 or more and 17 or less carbons. Examples of the hydrocarbon group include an alkyl group and an alkenyl group.
In the formula 1, each of the hydrocarbon groups of $R^1$ and $R^2$ has 6 or more, preferably 8 or more and more preferably 10 or more carbons from the viewpoint of softness, and 17 or less carbons from the viewpoint of dispersibility.
In the formula 1, the total number of carbons in $R^1$ and $R^2$ is 18 or more, preferably 20 or more, more preferably 21 or more and further preferably 22 or more from the viewpoint of softness, and 30 or less, preferably 28 or less, more preferably 26 or less and further preferably 25 or less from the viewpoint of dispersibility. Here, when the softening base agent contains two or more compounds having different total numbers of carbons in $R^1$ and $R^2$, the total number of carbons in $R^1$ and $R^2$ in the softening base agent represents the molar average of the total numbers of carbons in $R^1$ and $R^2$ of those compounds.
In the formula 1, the hydrocarbon groups of $R^1$ and $R^2$ may be either linear or branched, but preferably include a branched one from the viewpoint of dispersibility. In other words, the hydrocarbon groups of $R^1$ and $R^2$ preferably include a hydrocarbon group having a branched structure.
In the formula 1, the hydrocarbon groups of $R^1$ and $R^2$ may be either saturated or unsaturated, but preferably include an unsaturated one from the viewpoint of dispersibility. In other words, the hydrocarbon groups of $R^1$ and $R^2$ preferably include a hydrocarbon group having an unsaturated bond.
Accordingly, at least one of $R^1$ and $R^2$ in the formula 1 is a hydrocarbon group having a branched structure or an unsaturated bond.
In the formula 1, the hydrocarbon groups of $R^1$ and $R^2$ more preferably include a saturated branched one or an unsaturated linear one from the viewpoint of dispersibility. Each of the hydrocarbon groups of $R^1$ and $R^2$ is preferably a saturated branched one from the viewpoints of dispersion stability, and softness at a high hardness (for example, 20°DH), and preferably includes an unsaturated linear one from the viewpoints of dispersion stability, and softness at a medium hardness (for example, 8°DH).
In the formula 1, the hydrocarbon groups of $R^1$ and $R^2$ may be the same or different. In the method according to

the present invention, fibers are treated with a softening base agent comprising a compound represented by formula 1, in which the hydrocarbon groups of $R^1$ and $R^2$ are different. The hydrocarbon groups of $R^1$ and $R^2$ are preferably different from the viewpoint of softness at a high hardness. Further, the hydrocarbon groups of $R^1$ and $R^2$ are preferably the same from the viewpoints of dispersion stability, ease of production and softness in a low concentration. For example, $R^1$ and $R^2$ in the formula 1 may have the same or different numbers of carbons. $R^1$ and $R^2$ preferably have different numbers of carbons from the viewpoint of softness at a high hardness. Further, $R^1$ and $R^2$ preferably have the same number of carbons from the viewpoints of dispersion stability, ease of production and softness in a low concentration.

When the hydrocarbon groups of $R^1$ and $R^2$ in the formula 1 include a hydrocarbon group having a branched structure, the hydrocarbon groups of $R^1$ and $R^2$ each has preferably 1 or more and 2 or less, more preferably 1 or more and 1.5 or less, further preferably 1 or more and 1.2 or less, furthermore preferably 1 or more and 1.1 or less and furthermore preferably 1 branch from the viewpoints of softness and dispersion stability. Here, the number of branches is expressed by the number average of the numbers of branches in hydrocarbon groups having branched structures. Each of $R^1$ and $R^2$ preferably has 1 branch.

Each of the hydrocarbon groups of $R^1$ and $R^2$ having branched structures is preferably a hydrocarbon group having a branched chain in the second position, more preferably a hydrocarbon group having a branched chain with 2 or more carbons in the second position, further preferably an alkyl group having a branched chain with 2 or more carbons in the second position, furthermore preferably a hydrocarbon group having a branched chain derived from a Guerbet alcohol in the second position, and furthermore preferably a hydrocarbon group having a branched chain derived from a Guerbet alcohol only in the second position from the viewpoints of softness and dispersibility.

Note that, in the present invention, with the carbon of $R^1$ or $R^2$ bonded to O of $-O-R^1$ or $-O-R^2$ in the formula 1 defined as a carbon in the first position, a carbon bonded to the carbon in the first position is defined as a carbon in the second position, a carbon bonded to the carbon in the second position is defined as a carbon in the third position and a carbon bonded to the carbon in the third position is defined as a carbon in the fourth position, and the same applies to carbons in the fifth, sixth or subsequent positions. That is, the position of a carbon in $R^1$ and $R^2$ is determined by selecting the longest main chain including the carbon in the first position and determining the position of the carbon such as the second or subsequent positions on the basis of the main chain.

When the hydrocarbon groups of $R^1$ and $R^2$ in the formula 1 include a hydrocarbon group having a branched structure, the proportion of a hydrocarbon group having a branched structure at a carbon in the second or subsequent positions and only one methyl group bonded to the carbon in the second position (hereinafter also referred to as hydrocarbon group B2 of $R^1$ and $R^2$) is preferably 5 mol% or less, more preferably 4 mol% or less, further preferably 3 mol% or less, furthermore preferably 2 mol% or less, furthermore preferably 1 mol% or less and furthermore preferably 0 mol% in all the hydrocarbon groups of $R^1$ and $R^2$ from the viewpoint of softness.

Hydrocarbon group B2 of $R^1$ and $R^2$ is a hydrocarbon group represented by the formula 2:

$$-CH_2-CH(CH_3)-R^{21} \qquad \text{Formula 2}$$

wherein $R^{21}$ is a hydrocarbon group with 3 or more and 21 or less carbons.

$R^{21}$ is preferably an alkyl group, more preferably a linear alkyl group and further preferably a primary linear alkyl group from the viewpoint of availability.

$R^{21}$ in the formula 3 has preferably 5 or more and more preferably 6 or more carbons from the viewpoint of softness, and preferably 17 or less and more preferably 14 or less carbons from the viewpoint of dispersibility.

When the hydrocarbon groups of $R^1$ and $R^2$ in the formula 1 include a hydrocarbon group having a branched structure, the proportion of a hydrocarbon group having a branched structure only at a carbon in the third or subsequent positions and only one methyl group bonded to the carbon in the third position (hereinafter also referred to as hydrocarbon group B3 of $R^1$ and $R^2$) is preferably 10 mol% or less, more preferably 5 mol% or less, further preferably 2 mol% or less, furthermore preferably 1 mol% or less and furthermore preferably 0 mol% in all the hydrocarbon groups of $R^1$ and $R^2$ from the viewpoint of softness.

Hydrocarbon group B3 of $R^1$ and $R^2$ is a hydrocarbon group represented by the formula 3:

$$-R^{31}-CH(CH_3)-R^{32} \qquad \text{Formula 3}$$

wherein $R^{31}$ is a linear hydrocarbon group with 2 carbons, $R^{32}$ is a hydrocarbon group, and the total number of carbons in $R^{31}$ and $R^{32}$ is 4 or more and 22 or less.

$R^{31}$ is preferably an ethanediyl group from the viewpoint of availability, and $R^4$ is preferably an alkyl group, more preferably a linear alkyl group and further preferably a primary linear alkyl group from the viewpoint of availability.

The total number of carbons in $R^{31}$ and $R^{32}$ in the formula 3 is 4 or more, preferably 6 or more and more preferably

8 or more from the viewpoint of softness, and 22 or less, preferably 18 or less and more preferably 15 or less from the viewpoint of dispersibility.

When the hydrocarbon groups of $R^1$ and $R^2$ in the formula 1 include a hydrocarbon group having a branched structure, the proportion of a hydrocarbon group having a branched structure only at a carbon in the fourth or subsequent positions and only one methyl group bonded to the carbon at the smallest branch position (hereinafter also referred to as hydrocarbon group B4 of $R^1$ and $R^2$) is preferably 50 mol% or less, more preferably 40 mol% or less, further preferably 30 mol% or less, furthermore preferably 20 mol% or less, furthermore preferably 10 mol% or less, furthermore preferably 5 mol% or less, furthermore preferably 1 mol% or less and furthermore preferably 0 mol% in all the hydrocarbon groups of $R^1$ and $R^2$ from the viewpoint of softness.

Hydrocarbon group B4 of $R^1$ and $R^2$ is a hydrocarbon group represented by the formula 4:

$$-R^{41}-CH(CH_3)-R^{42} \qquad \text{Formula 4}$$

wherein $R^{41}$ is a linear hydrocarbon group with 3 or more carbons, $R^{42}$ is a hydrocarbon group, and the total number of carbons in $R^{41}$ and $R^{42}$ is 4 or more and 22 or less.
$R^{41}$ is preferably an alkane-α,ω-diyl group with 3 or more carbons from the viewpoint of availability, $R^{42}$ is preferably an alkyl group, more preferably a linear alkyl group and further preferably a primary saturated linear alkyl group from the viewpoint of availability.

The total number of carbons in $R^{41}$ and $R^{42}$ in the formula 4 is 4 or more, preferably 6 or more and more preferably 8 or more from the viewpoint of softness, and 22 or less, preferably 18 or less and more preferably 15 or less from the viewpoint of dispersibility.

The proportion of the hydrocarbon group having a branched structure at a carbon in the second or subsequent positions and only one methyl group bonded to the carbon in the second position, the proportion of the hydrocarbon group having a branched structure only at a carbon in the third or subsequent positions and only one methyl group bonded to the carbon in the third position, the proportion of the hydrocarbon group having a branched structure only at a carbon in the fourth or subsequent positions and only one methyl group bonded to the carbon at the smallest branch position or the like relative to all the hydrocarbon groups of $R^1$ and $R^2$ can each be measured by $^{13}$C-NMR after converting -O-$R^1$ and -O-$R^2$ respectively to alcohols H-O-$R^1$ and H-O-$R^2$ through hydrolysis or the like of the compound represented by the formula 1.

In results of the measurement by $^{13}$C-NMR under the conditions below, compound 1 has a proportion of the area of the region of signals in the range of 67.6 to 68 ppm relative to the area of the region of all signals between 60 and 69 ppm of preferably 5% or less, more preferably 4% or less, further preferably 3% or less, furthermore preferably 2% or less, furthermore preferably 1% or less and furthermore preferably 0%. Note that, in the following $^{13}$C-NMR, a chemical shift of the carbon in the first position can be determined from a plurality of branched alcohols having methyl branches as standard materials and utilized to predict chemical shifts in a sample.

$^{13}$C-NMR measurement conditions

Equipment: manufactured by Agilent Technologies, MR 400
Frequency: 400 MHz
Cumulative number: 1024
Latency time: 30 sec
Pulse angle: 45 deg
Deuterated solvent: $CDCl_3$
Sample concentration: 10%
Sample tube: 5 mmφ

In results of the measurement by $^{13}$C-NMR under the above conditions, compound 1 has a proportion of the area of the region of signals in the range of 60 to 61 ppm relative to the area of the region of all signals between 60 and 69 ppm of preferably 10% or less, more preferably 5% or less, further preferably 2% or less, furthermore preferably 1% or less and furthermore preferably 0%.

In results of the measurement by $^{13}$C-NMR under the above conditions, compound 1 has a proportion of the area of the region of signals in the range of 62 to 63.2 ppm relative to the area of the region of all signals between 60 and 69 ppm of preferably 50% or less, more preferably 40% or less, further preferably 30% or less, furthermore preferably 20% or less, furthermore preferably 10% or less, furthermore preferably 5% or less, furthermore preferably 1% or less and furthermore preferably 0%.

When the hydrocarbon groups of $R^1$ and $R^2$ in the formula 1 include a hydrocarbon group having a branched structure, the degree of branching of $R^1$ and $R^2$ that is defined by the following formula is preferably 0.3 or less, more preferably 0.2 or less and further preferably 0.1 or less from the viewpoint of softness, and preferably 0.01 or more, more preferably 0.02 or more and further preferably 0.04 or more from the viewpoint of dispersibility.

degree of branching=[(total number of terminal methyl groups in $R^1$ and $R^2$)-2]1/(total number of carbons in $R^1$ and $R^2$)

Note that, as the numbers of carbons used to calculate the degree of branching, average values of those measured by $^1$H-NMR can be used.

When the hydrocarbon groups of $R^1$ and $R^2$ in the formula 1 include a hydrocarbon group having an unsaturated bond, the unsaturated bond in the hydrocarbon groups of $R^1$ and $R^2$ is preferably a carbon-carbon double bond from the viewpoints of softness and dispersion stability.

When the hydrocarbon groups of $R^1$ and $R^2$ in the formula 1 include a hydrocarbon group having an unsaturated bond, the number of unsaturated bonds in the hydrocarbon groups of $R^1$ and $R^2$ is preferably 0.5 or more and 2 or less, more preferably 1 or more and 1.5 or less, further preferably 1 or more and 1.2 or less, furthermore preferably 1 or more and 1.1 or less, and furthermore preferably 1 from the viewpoints of softness, dispersion stability and availability. Here, the number of unsaturated bonds is expressed by the number average of the numbers of unsaturated bonds in hydrocarbon groups having unsaturated bonds.

When the hydrocarbon groups of $R^1$ and $R^2$ in the formula 1 include a hydrocarbon group having a double bond, the suitable range of the number of double bonds in the hydrocarbon groups of $R^1$ and $R^2$ is that of the number of unsaturated bonds.

Compound 1 or the compound represented by the formula 1 may be one or more compounds selected from a compound in which $R^1$ and $R^2$ are hydrocarbon groups having the same structure, and a compound in which $R^1$ and $R^2$ are hydrocarbon groups having different structures.

Compound 1 is preferably a compound in which $R^1$ and $R^2$ are hydrocarbon groups having different structures from the viewpoint of softness.

For example, the softening base agent of the present invention can contain a compound represented by the formula 1 in which $R^1$ and $R^2$ are hydrocarbon groups having the same structure, and a compound represented by the formula 1 in which $R^1$ and $R^2$ are hydrocarbon groups having different structures. The softening base agent of the present invention may contain as compound 1 the compound in which $R^1$ and $R^2$ are hydrocarbon groups having different structures.

M in the formula 1 is a cation, excluding a hydrogen ion. Examples of M include, for example, alkali metal ions such as a lithium ion, a sodium ion, a potassium ion and the like, alkaline earth metal ions such as a calcium ion, a barium ion and the like, organic ammonium ions such as a triethanolammonium ion, a diethanolammonium ion, a monoethanolammonium ion, a trimethylammonium ion, a monomethylammonium ion and the like, and others.

M is preferably an alkali metal ion or an alkanolammonium ion, more preferably a sodium ion, a potassium ion, a triethanolammonium ion, a diethanolammonium ion or a monoethanolammonium ion, and further preferably a sodium ion from the viewpoints of dispersion stability and softness.

Compound 1 can be synthesized by a publicly-known method. For example, a maleic acid diester obtained by reacting an alcohol with a maleic anhydrite can be reacted with a hydrogen sulfite to obtain compound 1. At that time, if alcohols different in carbon numbers or structures is used, compounds in which $R^1$ and $R^2$ in the formula 1 are hydrocarbon groups having different structures can be obtained. Compound 1 can be synthesized, for example, by the methods described in examples 2 to 3 of US-A 2007-0214999. Examples of the alcohol include, for example, a linear alcohol and an alcohol having a branch in the second position. Examples of the alcohol having a branch in the second position include a Guerbet alcohol.

The softening base agent of the present invention can be directed to various types of fibers, for example, natural fibers, synthetic fibers and semi-synthetic fibers. Further, the softening base agent of the present invention can be directed to textile products including these fibers.

The fibers may be either hydrophobic fibers or hydrophilic fibers. Examples of hydrophobic fibers include, for example, protein fibers (milk protein casein fiber, promix and the like), polyamide fibers (nylon and the like), polyester fibers (polyester and the like), polyacrylonitrile fibers (acrylic and the like), polyvinyl alcohol fibers (vinylon and the like), polyvinyl chloride fibers (polyvinyl chloride and the like), polyvinylidene chloride fibers (vinylidene and the like), polyolefin fibers (polyethylene, polypropylene and the like), polyurethane fibers (polyurethane and the like), polyvinyl chloride/polyvinyl alcohol copolymer fibers (polychlal and the like), polyalkylene paraoxybenzoate fibers (benzoate and the like), polyfluoroethylene fibers (polytetrafluoroethylene and the like) and others. Examples of hydrophilic fibers include, for example, seed hair fibers (cotton, cotton, kapok and the like), bast fibers (hemp, flax, ramie, cannabis, jute and the like), vein fibers

(manila hemp, sisal hemp and the like), palm fibers, juncus, straw, animal hair fibers (wool, mohair, cashmere, camel hair, alpaca, vicuna, angora and the like), silk fibers (domestic silk and wild silk), feathers, cellulose fibers (rayon, polynosic, cupro, acetate and the like) and others.

The fibers are preferably fibers including cotton fibers. The content of cotton fibers in the fibers is preferably 5 mass% or more, more preferably 10 mass% or more, further preferably 15 mass% or more, furthermore preferably 20 mass% or more and furthermore preferably 100 mass% from the viewpoint of further improving the softness of the fibers.

In the present invention, textile products mean woven fabrics, knitted fabrics, nonwoven fabrics and other fabrics using the above hydrophobic or hydrophilic fibers, and undershirts, T-shirts, dress shirts, blouses, slacks, caps, handkerchiefs, towels, knitwear, socks, underwear, tights and other products obtained from them. The textile products are preferably textile products including cotton fibers from the viewpoint of the effect of improving textures of fibers after treating with the fiber treatment agent composition of the present invention being more likely to be felt. Preferable modes of the content of cotton fibers in the textile products are the same as those of the content of cotton fibers in the fibers.

The softening base agent of the present invention can impart softness to fibers. Further, the softening base agent of the present invention is also excellent in dispersibility when mixed with water.

The present invention discloses the use of compound 1 represented by the formula 1 as a softening base agent. The matters mentioned in the softening base agent, the below-mentioned fiber treatment agent composition and method for treating fibers of the present invention can be appropriately applied to the use of the present invention. Specific examples or preferable modes of compound 1 or the like are also the same as those in the softening base agent of the present invention.

[Fiber treatment agent composition]

The present invention provides a fiber treatment agent composition containing the softening base agent of the present invention. The matters mentioned in the softening base agent of the present invention can be appropriately applied to the fiber treatment agent composition of the present invention. Specific examples or preferable modes of compound 1 or the like are also the same as those in the softening base agent of the present invention.

The content of compound 1 in the fiber treatment agent composition of the present invention is preferably 1 mass% or more, more preferably 2 mass% or more and further preferably 4 mass% or more from the viewpoint of transportability, and preferably 90 mass% or less, more preferably 70 mass% or less and further preferably 50 mass% or less from the viewpoint of handleability.

The fiber treatment agent composition of the present invention can also contain a softening base agent other than compound 1 (hereinafter also referred to as optional softening base agent), but the content thereof may be small. The content of the optional softening base agent in the fiber treatment agent composition of the present invention may be, for example, less than 1 mass% and further less than 0.1 mass% in the composition from the viewpoints of softness, dispersibility and water absorbency. Further, the mass ratio of (content of optional softening base agent)/(content of compound 1) in the fiber treatment agent composition of the present invention may be, for example, 4.5 or less, further 4.0 or less, further 3.0 or less, further 2.0 or less, further 1.0 or less, further 0.50 or less, further 0.30 or less, further less than 0.10 and further 0.050 or less from the same viewpoints. Here, examples of the optional softening base agent include, for example, a cationic softening agent and a nonionic softening base agent. The cationic softening base agent can be selected from, for example, quaternary ammonium salts. The nonionic softening base agent can be selected from, for example, a higher fatty acid ester of pentaerythritol, an oligomer of pentaerythritol, a lower alkylene oxide derivative of pentaerythritol, a lower alkylene oxide derivative of an oligomer of pentaerythritol and the like.

The fiber treatment agent composition of the present invention preferably contains water. The fiber treatment agent composition of the present invention is preferably a liquid composition containing water. Water is usually the balance of the composition and used in such an amount that makes the total of the composition 100 mass%.

When the fiber treatment agent composition of the present invention contains water, the proportion of compound 1 in the total of components other than water may be, for example, 20 mass% or more, further 30 mass% or more, further 40 mass% or more, further 50 mass% or more, further 60 mass% or more, further 70 mass% or more, further 80 mass% or more, further 90 mass% or more, further 92 mass% or more and further 95 mass% or more. The upper limit may be 100 mass% or less.

The fiber treatment agent composition of the present invention may have a pH at 20°C of, for example, 4.0 or more, further 5.0 or more, further 5.5 or more, further 6.0 or more and further 7.0 or more.

The fiber treatment agent composition of the present invention may be a softening agent composition. For example, the present invention can provide a softening agent composition containing compound 1 as an active ingredient of the softening base agent.

As compound 1 has good dispersibility in water, the fiber treatment agent composition of the present invention containing water can be produced by mixing compound 1 with water at a relatively low temperature. The temperature of water to be mixed with compound 1 may be, for example, 30°C or more and 50°C or less. According to the present

invention, provided is a method for producing a fiber treatment agent composition including, mixing compound 1 with water at 30°C or more and 50°C or less.

[Method for treating fibers]

The present invention provides a method for treating fibers including, treating the fibers with the softening base agent of the present invention as outlined in appended claim 1, wherein compound 1 is used in an amount of 0.01% o.w.f. or more and 5% o.w.f. or less relative to the fibers. Further embodiments are subject to the dependent claims. Further, the present invention may be a method for treating fibers including, treating the fibers with the softening base agent, wherein compound 1 is used in an amount of 0.05% o.w.f. or more and 5% o.w.f. or less relative to the fibers. The matters mentioned in the softening base agent and the fiber treatment agent composition of the present invention can be appropriately applied to the method for treating fibers of the present invention. Specific examples or preferable modes of compound 1 or the like are also the same as those in the softening base agent of the present invention. The method for treating fibers of the present invention may be a method for treating fibers, wherein compound 1 is applied to the fibers in an amount of 0.01% o.w.f. or more and 5% o.w.f. or less to impart softness to the fibers. Further, the method for treating fibers of the present invention may be a method for treating fibers, wherein compound 1 is applied to the fibers in an amount of 0.05% o.w.f. or more and 5% o.w.f. or less to impart softness to the fibers. The fiber treatment agent composition of the present invention can be used in the method for treating fibers of the present invention.

In the present invention, compound 1 is used in an amount of 0.01% o.w.f. or more, preferably 0.05% o.w.f. or more, more preferably 0.1% o.w.f. or more, further preferably 0.2% o.w.f. or more and furthermore preferably 0.3% o.w.f. or more relative to the fibers from the viewpoint of softness, and 5% o.w.f. or less, preferably 4% o.w.f. or less, more preferably 3% o.w.f. or less and further preferably 2% o.w.f. or less relative to the fibers from the viewpoint of textures. Note that % o.w.f. is an abbreviation of % on the weight of fabric, and means the percentage of the mass of compound 1 relative to the mass of fibers. In the present invention, a treatment liquid obtained by mixing the softening base agent of the present invention or the fiber treatment agent composition of the present invention with water can be brought into contact with the fibers. For example, the treatment liquid can be used such that the amount of compound 1 relative to the fibers falls within the above range.

In the present invention, the softening base agent is preferably used by mixing with water having a hardness of 0°DH or more and 30°DH or less. That is, the fibers are preferably treated with a treatment liquid obtained by mixing the softening base agent with water having a hardness of 0°DH or more and 30°DH or less. The hardness of water is preferably 1°DH or more, more preferably 2°DH or more and further preferably 3°DH or more from the viewpoint of softness, and preferably 25°DH or less and more preferably 20°DH or less from the viewpoint of textures.

The method for treating fibers of the present invention can be directed to the fibers mentioned in the softening base agent of the present invention. For example, the fibers may be fibers of fabric.

The method for treating fibers of the present invention can be performed by incorporating into washing processes of fibers, for example, fibers of fabric. Here, the washing processes may be treatments such as washing, rinsing and dewatering fibers. In the present invention, the softening base agent of the present invention can be applied to fibers in any of these washing processes such that compound 1 is used in a predetermined amount.

Examples

<Production examples 1 to 8>

A dialkyl sulfosuccinate of each production example listed in Table 1 was prepared as follows. In the reaction vessel shown in Table 1 equipped with a stirrer, a heating system, a distillation column and a nitrogen/vacuum connection, raw materials and the catalyst listed in Table 1 used to prepare a maleic acid diester were prepared in their respective amounts shown in Table 1, and after nitrogen substitution, reacted under nitrogen bubbling while dewatered at 100 to 130°C until an acid value was lowered to an amount equivalent to that of p-toluenesulfonic acid. Subsequently, the catalyst was adsorbed to KYOWAAD® 500SH (manufactured by Kyowa Chemical Industry Co., Ltd.) in an amount of 1 mass% relative to the total amount of the contents in the reaction vessel. After removing the adsorbent, an excess alcohol was removed by topping, thereby obtaining a maleic acid diester.

Next, in a 1-L glass reaction vessel, the maleic acid diester obtained above, sodium disulfite and ion exchange water were prepared in their respective amounts shown in Table 1, and using an alcohol polar solvent such as ethanol or the like to improve the compatibility of raw materials used to prepare each dialkyl sulfosuccinate listed in Table 1, reacted at 115°C by a publicly-known method until it was confirmed by NMR that a double bond derived from the maleic acid diester disappeared. The reaction product was cooled to 50 to 65°C and the remaining sodium hydrogen sulfite was oxidized with 30% hydrogen peroxide, and thereafter the pH was adjusted to 5 with 10% NaOH. The solvent and sodium sulfate were removed by distillation under reduced pressure, reprecipitation, liquid separation or the like, thereby obtaining each dialkyl

sulfosuccinate listed in Table 1.

[Table 1]

| | | | | Production example 1 | Production example 2 | Production example 3 | Production example 4 | Production example 5 | Production example 6 | Production example 7 | Production example 8 |
|---|---|---|---|---|---|---|---|---|---|---|---|
| Dialkyl sulfosuccinate | | | | Di(2-propylheptyl)-sulfosuccinate | Di(2-butyloctyl)-sulfosuccinate | Dodecyl/2-butyloctyl-sulfosuccinate | Octyl/cetyl-sulfosuccinate | Dodecyl/3-nonenyl-sulfosuccinate | Dicetyl sulfosuccinate | Stearyl/oleyl-sulfosuccinate | Cetyl/stearyl sulfosuccinate |
| Preparation of maleic acid diester | Reaction vessel | Type | | Four neck flask | Four neck flask | Four neck flask | Four neck flask | Four neck flask | Four neck flask | Four neck flask | Four neck flask |
| | | Capacity | | 2L | 2L | 500 mL | 500 mL | 500 mL | 2L | 500 mL | 500 mL |
| | Raw material | Maleic anhydride | Preparation amount (g) | 176.5 g | 176.5 g | 58.8 g | 58.8 g | 68.6 g | 138 g | 49.0 g | 49.0 g |
| | | | Preparation amount (mol) | 1.8 mol | 1.8 mol | 0.60 mol | 0.60 mol | 0.70 mol | 1.4 mol | 0.50 mol | 0.50 mol |
| | | Alcohol 1 | Type | 2-propylheptanol | 2-butyloctanol | 2-butyloctanol | Octanol | Dodecanol | Cetanol | Oleyl alcohol | Cetanol |
| | | | Preparation amount (g) | 626.6 g | 737.8 g | 123.0 g | 86.0 g | 143.5 g | 749 g | 147.7 g | 133.3 g |
| | | | Preparation amount (mol) | 4.0 mol | 4.0 mol | 0.66 mol | 0.66 mol | 0.77 mol | 3.1 mol | 0.55 mol | 0.55 mol |
| | | Alcohol 2 | Type | - | - | Dodecanol | Cetanol | Cis-3-nonene-1-ol | - | Stearyl alcohol | Stearyl alcohol |
| | | | Preparation amount (g) | - | - | 123.0 g | 160.0 g | 109.5 g | - | 148.8 g | 148.8 g |
| | | | Preparation amount (mol) | - | - | 0.66 mol | 0.66 mol | 0.77 mol | - | 0.55 mol | 0.55 mol |
| | Catalyst | p-toluenesulfonic acid monohydrate | Preparation amount (g) | 2.5 g | 2.5 g | 0.82 g | 0.82 g | 0.84 g | 27 g | 0.98 g | 0.93 g |
| | | | Preparation amount (mol) | 0.013 mol | 0.013 mol | 0.0043 mol | 0.0043 mol | 0.0044 mol | 0.014 mol | 0.0052 mol | 0.0049 mol |

(continued)

| | | | | Production example 1 | Production example 2 | Production example 3 | Production example 4 | Production example 5 | Production example 6 | Production example 7 | Production example 8 |
|---|---|---|---|---|---|---|---|---|---|---|---|
| Preparation of dialkyl sulfosuccinate | Raw material | Maleic acid diester | Preparation amount (g) | 278 g | 200 g | 150 g | 150 g | 150 g | 200 g | 150 g | 150 g |
| | | | Preparation amount (mol) | 0.70 mol | 0.44 mol | 0.33 mol | 0.31 mol | 0.37 mol | 0.35 mol | 0.24 mol | 0.25 mol |
| | | Sodium disulfite | Preparation amount (g) | 73 g | 46 g | 35 g | 32 g | 38 g | 37 g | 25g | 26 g |
| | | | Preparation amount (mol) | 0.38 mol | 0.24 mol | 0.18 mol | 0.77 mol | 0.20 mol | 0.19 mol | 0.13 mol | 0.14 mol |
| | | Ion exchange water | Preparation amount (g) | 48 g | 18 g | 13 g | 12 g | 14 g | 18 g | 9.6 g | 10 g |
| | | | Preparation amount (mol) | 2.7 mol | 1.0 mol | 0.73 mol | 0.68 mol | 0.81 mol | 1.0 mol | 0.53 mol | 0.56 mol |

The components in Table 1 are listed below.

Maleic anhydride: manufactured by FUJIFILM Wako Pure Chemical Corporation, Wako Special Grade
2-propylheptanol: manufactured by FUJIFILM Wako Pure Chemical Corporation, Guaranteed Reagent
2-butyloctanol: 2-butyl-1-n-octanol, manufactured by FUJIFILM Wako Pure Chemical Corporation, Guaranteed Reagent
Octanol: manufactured by Kao Corporation, "KALCOL 0898"
Dodecanol: manufactured by Kao Corporation, "KALCOL 2098"
Cetanol: manufactured by Kao Corporation, "KALCOL 6098"
Oleyl alcohol: manufactured by Alfa Aesar
Cis-3-nonene-1-ol: manufactured by Tokyo Chemical Industry Co., Ltd.
Stearyl alcohol: manufactured by Kao Corporation, "KALCOL 8098"
P-toluenesulfonic acid monohydrate: manufactured by FUJIFILM Wako Pure Chemical Corporation, Guaranteed Reagent
Sodium disulfite: manufactured by FUJIFILM Wako Pure Chemical Corporation, Guaranteed Reagent

<Examples 1 to 5 and comparative examples 1 to 5>

Using the dialkyl sulfosuccinates listed in Table 1 as softening base agents, softness and the stability of dispersions were evaluated in the following manner. The results are shown in Table 2. Note that Table 2 shows the structures in the formula 1 of the softening base agents. For compounds that do not qualify as compound 1, structures corresponding to those in the formula 1 are shown for convenience.

The softening base agents listed in Table 2 are as follows.

· Reference product 1: di(2-propylheptyl)-sulfosuccinate prepared in production example 1
· Reference product 2: di(2-butyloctyl)-sulfosuccinate prepared in production example 2
· Inventive product 3: dodecyl/2-butyloctyl-sulfosuccinate prepared in production example 3
· Reference product 4: octyl/cetyl-sulfosuccinate prepared in production example 4
· Inventive product 5: dodecyl/3-nonenyl-sulfosuccinate prepared in production example 5
· Comparative product 1: di(2-ethylhexyl)sulfosuccinate, reagent, DIOCTYLSULFOSUCCINATE (MP Biomedical, Inc.)
· Comparative product 2: dicetyl sulfosuccinate prepared in production example 6
· Comparative product 3: stearyl/oleyl-sulfosuccinate prepared in production example 7
· Comparative product 4: cetyl/stearyl-sulfosuccinate prepared in production example 8
· Comparative product 5: $\alpha$-olefin sulfonate, LIPOLAN PB-800CJ, manufactured by Lion Corporation

· Method for evaluating softness

1) Pretreatment of towel used for evaluation

Towels from which sizing agents or impurities were removed by the following pretreatment were used for evaluation.

In a fully automatic washing machine (manufactured by Panasonic Corporation, Model No.: NA-F60PB3), 52.22 g of a 10% diluent of a nonionic surfactant (manufactured by Kao Corporation, EMULGEN 108) was added as a detergent to 24 commercially available cotton towels (manufactured by Takei Towel Co., Ltd., TW220, white), and a series of washing processes (water volume 50 L, washing for 10 minutes-water-saving rinsing twice-dewatering for 9 minutes) were repeated 3 times using tap water of Wakayama city (the tap water is water having a hardness of 4°DH, and the same applies hereinafter) as water. Subsequently, the series of washing processes were repeated twice using only water. After that, the towels were left and naturally dried at room temperature (25°C) for 24 hours.

2) Method for treating towel

In National MiniMini Washer NA-35, a predetermined amount of ion exchange water was placed (such that the bath ratio was 25 liters per kilogram of towels) and an aqueous calcium chloride solution (equivalent to 4000°DH) was added to make the hardness 20°DH, and while stirring them, a 5 mass% water dispersion of each softening base agent in Table 2 was added and stirred for 1 minute, and thereafter, 3 cotton towels (about 210 g in total) pretreated in the above 1) were placed therein and treated for 5 minutes under stirring. In this treatment, each softening base agent in Table 2 was used in an amount of 0.5% o.w.f. with reference to 3 cotton towels. Subsequently, the cotton towels were dewatered for 3

minutes in a dewatering tank of a two-tank washing machine (manufactured by TOSHIBA CORPORATION, Model No.: VH-52G(H)), and dried in a thermo-hygrostat at 23°C and 40%RH for 24 hours. Similarly, an aqueous calcium chloride solution was added to make the hardness 8°DH and a 5 mass% water dispersion of each softening base agent was added in an amount of 0.3% o.w.f. to obtain a treatment liquid, and cotton towels were treated therewith and dried.

3) Softness evaluation

A cotton towel treated with a formulation indicated in each score below was prepared as a reference by the methods in the above 1) and 2).

The softness of a cotton towel treated with each softening base agent listed in Table 2 was compared with that of each reference cotton towel to evaluate the softness. Scores (points) given by five panelists in accordance with the following criteria to make evaluations were averaged and listed in the table. Note that the scores given by the panelists to make evaluations could be decimal fraction values between values of two scores.

Score 1: as soft as that treated only with tap water at 20°C
Score 2: as soft as that treated with a formulation using an indicator softening base agent in an amount of 0.025% o.w.f. in tap water at 20°C
Score 3: as soft as that treated with a formulation using the indicator softening base agent in an amount of 0.050% o.w.f. in tap water at 20°C
Score 4: as soft as that treated with a formulation using the indicator softening base agent in an amount of 0.075% o.w.f. in tap water at 20°C
Score 5: as soft as that treated with a formulation using the indicator softening base agent in an amount of 0.100% o.w.f. in tap water at 20°C

Here, an ester amide hydrochloride (2-[N-[3-alkanoyl(C14-20)aminopropyl]-N-methylamino]ethylalkano(C14-20)ate hydrochloride) was used as the indicator softening base agent.

· Method for evaluating dispersion stability

5 g of each softening base agent in Table 2 and 95 g of ion exchange water were mixed and stirred at 80°C for 20 minutes, and thereafter stirred at room temperature (20°C) for 20 minutes, and left at room temperature for 24 hours. After that, the mixture was further left at 5°C for 24 hours. The dispersion stability was evaluated by observing the appearance of the mixture. Evaluation criteria for the dispersion stability were shown below. The dispersion stability was evaluated on the basis of the appearance observed at room temperature immediately after stirring at 80°C for 20 minutes (immediately after preparation) and the appearance observed at a liquid temperature of 5°C after leaving at 5°C for 24 hours (after leaving at 5°C for 24 hours).

*Evaluation criteria for dispersion stability

Good: no precipitations
Average: a slight precipitation
Poor: a large amount of precipitation

[Table 2]

| | | | Example 1 | Example 2 | Example 3 | Example 4 | Example 5 |
|---|---|---|---|---|---|---|---|
| Softening base agent | Compound | | Inventive product 1 | Inventive product 2 | Inventive product 3 | Inventive product 4 | Inventive product 5 |
| | Structure in formula 1 | $R^1$, $R^2$ | 2-propylheptyl (branched saturated) | 2-butyloctyl (branched saturated) | 2-butyloctyl (branched saturated): dodecyl (linear saturated) =1:1 (molar ratio) | Octyl (linear saturated): cetyl (linear saturated) =1:1 (molar ratio) | Dodecyl (linear saturated): 3-nonenyl (linear unsaturated) =1:1 (molar ratio) |
| | | Total number of carbons in $R^1$ and $R^2$ (molar average) | 20 | 24 | 24 | 24 | 21 |
| | | M | Na | Na | Na | Na | Na |
| | | Degree of branching | 0.1 | 0.083 | 0.042 | 0 | 0 |
| Dispersion stability | Immediately after preparation | | Good | Good | Good | Good | Good |
| | After leaving for 24 hours at 5°C | | Good | Good | Good | Average | Good |
| Softness (point) | 20°DH | | 3.2 | 2.9 | 4.2 | 4.7 | 3.4 |
| | 8°DH | | 2.8 | 2.5 | 2.5 | 3.1 | 2.6 |

| | | | Comparative example 1 | Comparative example 2 | Comparative example 3 | Comparative example 4 | Comparative example 5 |
|---|---|---|---|---|---|---|---|
| Softening base agent | Compound | | Comparative product 1 | Comparative product 2 | Comparative product 3 | Comparative product 4 | Comparative product 5 |
| | Structure in formula 1 | $R^1$, $R^2$ | 2-ethylhexyl (branched saturated) | Cetyl (linear saturated) | Stearyl (linear saturated): oleyl (linear unsaturated) =1:1 (molar ratio) | Cetyl (linear saturated): stearyl (linear saturated) =1:1 (molar ratio) | — |
| | | Total number of carbons in $R^1$ and $R^2$ (molar average) | 16 | 32 | 36 | 34 | — |
| | | M | Na | Na | Na | Na | — |
| | | Degree of branching | 0.125 | 0 | 0 | 0 | — |
| Dispersion stability | Immediately after preparation | | Good | Good | Poor | Good | Good |
| | After leaving for 24 hours at 5°C | | Good | Average | Poor | Poor | Good |
| Softness (point) | 20°DH | | 1.7 | 2.3 | Not evaluable | 1.8 | 1.7 |
| | 8°DH | | 1.9 | 2.4 | 2.4 | 1.7 | 1.2 |

From the results in Table 2 showing that the inventive softening base agents impart higher softness than the comparative softening base agents, it can be seen that the inventive softening base agents are softening base agents superior to other anionic surfactants in fiber softening effect. Further, it can be seen that the inventive softening base agents exhibit more excellent softness when used with water having a higher hardness. In addition, it can be seen that the inventive softening base agents have excellent dispersion stability in water.

## Claims

**1.** A method for treating fibers comprising, treating the fibers with a softening base agent comprising a compound represented by the following formula 1, wherein the compound represented by the formula 1 is used in an amount of 0.01% o.w.f. or more and 5% o.w.f. or less relative to the fibers:

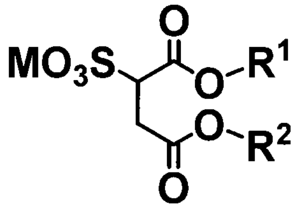

Formula 1

wherein each of $R^1$ and $R^2$ represents a hydrocarbon group with 6 or more and 17 or less carbons, and the total number of carbons in $R^1$ and $R^2$ is 18 or more and 30 or less; and M is a cation, excluding a hydrogen ion;
wherein at least one of $R^1$ and $R^2$ in the formula 1 is a hydrocarbon group having a branched structure or an unsaturated bond;
wherein the hydrocarbon groups of $R^1$ and $R^2$ are different; and
wherein the hydrocarbon group having a branched structure has a branched chain in the second position, and the branched chain is a hydrocarbon group with 2 or more carbons, wherein the second position is defined as a carbon of $R^1$ or $R^2$ bonded to the carbon in a first position, wherein the first position is defined as a carbon of $R^1$ or $R^2$ bonded to O of $-O-R^1$ or $-O-R^2$ in formula 1.

2. The method according to claim 1, wherein the agent comprises a compound represented by the formula 1 in which $R^1$ and $R^2$ are hydrocarbon groups having the same structure, and a compound represented by the formula 1 in which $R^1$ and $R^2$ are hydrocarbon groups having different structures.

3. The method according to claims 1 or 2, wherein the hydrocarbon groups of $R^1$ and $R^2$ in the formula 1 comprise a hydrocarbon group having a branched structure, and a proportion of hydrocarbon groups of $R^1$ and $R^2$ represented by the following formula 4 is 50 mol% or less in all the hydrocarbon groups of $R^1$ and $R^2$,

$$-R^{41}-CH(CH_3)-R^{42} \qquad \text{Formula 4}$$

wherein $R^{41}$ is a linear hydrocarbon group with 3 or more carbons, $R^{42}$ is a hydrocarbon group, and the total number of carbons in $R^{41}$ and $R^{42}$ is 4 or more and 22 or less.

4. The method according to any one of claims 1 to 3, wherein the fibers are treated with a treatment liquid obtained by mixing the softening base agent with water having a hardness of 0°DH or more and 30°DH or less.

5. The method according to any one of claims 1 to 4, wherein the fibers are fabric.

6. The method according to any one of claims 1 to 5, wherein the fibers are treated in a washing process.

7. Use of a compound represented by the following formula 1 as a softening base agent for treating fibers:

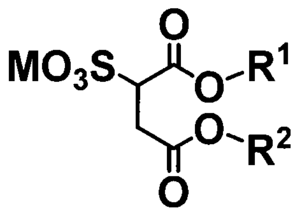

Formula 1

wherein each of $R^1$ and $R^2$ represents a hydrocarbon group with 6 or more and 17 or less carbons, and the total number of carbons in $R^1$ and $R^2$ is 18 or more and 30 or less; and M is a cation, excluding a hydrogen ion,
wherein at least one of $R^1$ and $R^2$ in the formula 1 is a hydrocarbon group having a branched structure or an unsaturated bond;
wherein the hydrocarbon groups of $R^1$ and $R^2$ are different; and
wherein the hydrocarbon group having a branched structure has a branched chain in the second position, and the branched chain is a hydrocarbon group with 2 or more carbons, wherein the second position is defined as a carbon of $R^1$ or $R^2$ bonded to the carbon in a first position, wherein the first position is defined as a carbon of $R^1$ or $R^2$ bonded to O of $-O-R^1$ or $-O-R^2$ in formula 1.

**Patentansprüche**

1. Verfahren zur Behandlung von Fasern, umfassend die Behandlung der Fasern mit einem Weichmacher umfassend eine Verbindung der folgenden Formel 1, wobei die durch die Formel 1 dargestellte Verbindung in einer Menge von 0,01% o.w.f. (bezogen auf das Fasergewicht) oder mehr und 5% o.w.f. oder weniger, bezogen auf die Fasern, verwendet wird:

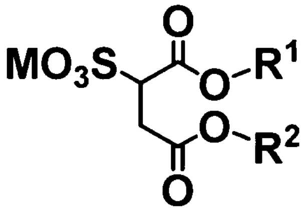

Formel 1

wobei $R^1$ und $R^2$ jeweils eine Kohlenwasserstoffgruppe mit 6 oder mehr und 17 oder weniger Kohlenstoffatomen darstellen, und die Gesamtzahl der Kohlenstoffatome in $R^1$ und $R^2$ 18 oder mehr und 30 oder weniger beträgt; und M ein Kation ist, mit Ausnahme eines Wasserstoffions;
wobei mindestens einer von $R^1$ und $R^2$ in der Formel 1 eine Kohlenwasserstoffgruppe mit einer verzweigten Struktur oder einer ungesättigten Bindung ist;
wobei die Kohlenwasserstoffgruppen von $R^1$ und $R^2$ unterschiedlich sind; und
wobei die Kohlenwasserstoffgruppe mit einer verzweigten Struktur eine verzweigte Kette in der zweiten Position aufweist, und die verzweigte Kette eine Kohlenwasserstoffgruppe mit 2 oder mehr Kohlenstoffatomen ist, wobei die zweite Position als ein Kohlenstoffatom von $R^1$ oder $R^2$ definiert ist, das an das Kohlenstoffatom in einer ersten Position gebunden ist, wobei die erste Position als ein Kohlenstoffatom von $R^1$ oder $R^2$ definiert ist, das an O von $-O-R^1$ oder $-O-R^2$ in Formel 1 gebunden ist.

2. Verfahren gemäß Anspruch 1, wobei das Mittel umfasst eine Verbindung der Formel 1, in der $R^1$ und $R^2$ Kohlenwasserstoffgruppen mit derselben Struktur sind, und eine Verbindung der Formel 1, in der $R^1$ und $R^2$ Kohlenwasserstoffgruppen mit unterschiedlichen Strukturen sind.

3. Verfahren gemäß Anspruch 1 oder 2, wobei die Kohlenwasserstoffgruppen von $R^1$ und $R^2$ in Formel 1 umfassen eine Kohlenwasserstoffgruppe mit einer verzweigter Struktur, und der Anteil der durch die folgende Formel 4 dargestellten Kohlenwasserstoffgruppen von $R^1$ und $R^2$ an den gesamten Kohlenwasserstoffgruppen von $R^1$ und $R^2$ 50 Mol-% oder weniger beträgt:

$$-R^{41}-CH(CH_3)-R^{42} \qquad \text{Formel 4}$$

wobei $R^{41}$ eine lineare Kohlenwasserstoffgruppe mit 3 oder mehr Kohlenstoffatomen ist, $R^{42}$ eine Kohlenwasserstoffgruppe ist, und die Gesamtzahl der Kohlenstoffatome in $R^{41}$ und $R^{42}$ 4 oder mehr und 22 oder weniger beträgt.

4. Verfahren gemäß mindestens einem der Ansprüche 1 bis 3, wobei die Fasern mit einer Behandlungsflüssigkeit behandelt werden, die durch Mischen des Weichmachers mit Wasser mit einer Härte von 0 °DH oder mehr und 30 °DH oder weniger erhalten wird.

5. Verfahren gemäß mindestens einem der Ansprüche 1 bis 4, wobei die Fasern Stoff sind.

6. Verfahren gemäß mindestens einem der Ansprüche 1 bis 5, wobei die Fasern in einem Waschprozess behandelt werden.

7. Verwendung einer Verbindung der folgenden Formel 1 als Weichmacher zur Behandlung von Fasern:

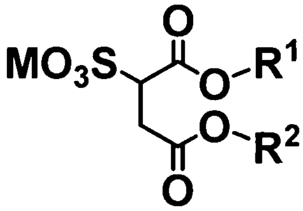

Formel 1

wobei $R^1$ und $R^2$ jeweils eine Kohlenwasserstoffgruppe mit 6 oder mehr und 17 oder weniger Kohlenstoffatomen darstellen, und die Gesamtzahl der Kohlenstoffatome in $R^1$ und $R^2$ 18 oder mehr und 30 oder weniger beträgt; und M ein Kation ist, mit Ausnahme eines Wasserstoffions,

wobei mindestens einer von $R^1$ und $R^2$ in Formel 1 eine Kohlenwasserstoffgruppe mit einer verzweigten Struktur oder einer ungesättigten Bindung ist;

wobei die Kohlenwasserstoffgruppen von $R^1$ und $R^2$ unterschiedlich sind; und

wobei die Kohlenwasserstoffgruppe mit einer verzweigten Struktur eine verzweigte Kette in der zweiten Position aufweist, und die verzweigte Kette eine Kohlenwasserstoffgruppe mit 2 oder mehr Kohlenstoffatomen ist, wobei die zweite Position als ein Kohlenstoffatom von $R^1$ oder $R^2$ definiert ist, das an das Kohlenstoffatom in einer ersten Position gebunden ist, wobei die erste Position als ein Kohlenstoffatom von $R^1$ oder $R^2$ definiert ist, das an O von $-O-R^1$ oder $-O-R^2$ in Formel 1 gebunden ist.

**Revendications**

1. Procédé pour le traitement de fibres comprenant, le traitement des fibres avec un agent de base assouplissant comprenant un composé représenté par la formule 1 suivante, dans laquelle le composé représenté par la formule 1 est utilisé en une quantité de 0,01 % sur poids d'étoffe ou plus et de 5 % sur poids d'étoffe ou moins par rapport aux fibres :

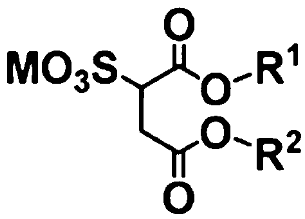

Formule 1

dans laquelle chacun de $R^1$ et de $R^2$ représente un groupe hydrocarboné ayant 6 atomes de carbone ou plus et 17 atomes de carbone ou moins, et le nombre total d'atomes de carbone dans $R^1$ et $R^2$ est de 18 ou plus et de 30 ou moins ; et M est un cation, à l'exclusion d'un ion hydrogène ;

dans laquelle au moins un de $R^1$ et de $R^2$ dans la formule 1 est un groupe hydrocarboné ayant une structure ramifiée ou une liaison insaturée ;

dans lequel les groupes hydrocarbonés de $R^1$ et de $R^2$ sont différents; et

dans lequel le groupe hydrocarboné ayant une structure ramifiée a une chaîne ramifiée dans la deuxième position, et la chaîne ramifiée est un groupe hydrocarboné ayant 2 atomes de carbone ou plus, dans lequel la deuxième position est définie comme étant un atome de carbone de $R^1$ ou de $R^2$ lié à l'atome de carbone dans une première position, dans lequel la première position est définie comme étant un atome de carbone de $R^1$ ou de $R^2$ lié au 0 de $-O-R^1$ ou de $-O-R^2$ dans la formule 1.

2. Procédé selon la revendication 1, dans lequel l'agent comprend un composé représenté par la formule 1 dans laquelle $R^1$ et $R^2$ sont des groupes hydrocarbonés ayant la même structure, et un composé représenté par la formule 1 dans laquelle $R^1$ et $R^2$ sont des groupes hydrocarbonés ayant des structures différentes.

3. Procédé selon la revendication 1 ou la revendication 2, dans lequel les groupes hydrocarbonés de $R^1$ et de $R^2$ dans la formule 1 comprennent un groupe hydrocarboné ayant une structure ramifiée, et une proportion des groupes hydrocarbonés de $R^1$ et de $R^2$ représentés par la formule 4 suivante est de 50 mol% ou moins dans tous les groupes hydrocarbonés de $R^1$ et de $R^2$,

$-R^{41}-CH(CH_3)-R^{42}$ Formule 4

dans lequel $R^{41}$ est un groupe hydrocarboné linéaire ayant 3 atomes de carbone ou plus, $R^{42}$ est un groupe hydrocarboné, et le nombre total d'atomes de carbone dans $R^{41}$ et $R^{42}$ est de 4 ou plus et de 22 ou moins.

**4.** Procédé selon l'une quelconque des revendications 1 à 3, dans lequel les fibres sont traitées avec un liquide de traitement obtenu par mélange de l'agent de base assouplissant avec de l'eau ayant une dureté de 0°DH ou plus et de 30°DH ou moins.

**5.** Procédé selon l'une quelconque des revendications 1 à 4, dans lequel les fibres sont une étoffe.

**6.** Procédé selon l'une quelconque des revendications 1 à 5, dans lequel les fibres sont traitées dans un procédé de lavage.

**7.** Utilisation d'un composé représenté par la formule 1 suivante en tant qu'agent de base assouplissant pour le traitement de fibres :

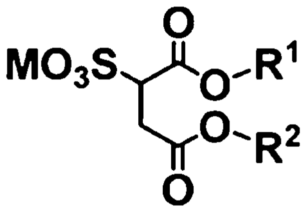

Formule 1

dans lequel chacun de $R^1$ et de $R^2$ représente un groupe hydrocarboné ayant 6 atomes de carbone ou plus et 17 atomes de carbone ou moins, et le nombre total d'atomes de carbone dans $R^1$ et $R^2$ est de 18 ou plus et de 30 ou moins ; et M est un cation, à l'exclusion d'un ion hydrogène,
dans lequel au moins un de $R^1$ et de $R^2$ dans la formule 1 est un groupe hydrocarboné ayant une structure ramifiée ou une liaison insaturée ;
dans lequel les groupes hydrocarbonés de $R^1$ et de $R^2$ sont différents ; et
dans lequel le groupe hydrocarboné ayant une structure ramifiée a une chaîne ramifiée dans la deuxième position, et la chaîne ramifiée est un groupe hydrocarboné ayant 2 atomes de carbone ou plus, dans lequel la deuxième position est définie comme étant un atome de carbone de $R^1$ ou de $R^2$ lié à l'atome de carbone dans une première position, dans lequel la première position est définie comme étant un atome de carbone de $R^1$ ou de $R^2$ lié au 0 de $-O-R^1$ ou de $-O-R^2$ dans la formule 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005171399 A
- JP H8158258 A
- US 5419842 B
- US 4176080 A
- JP H0827662 A
- JP 2012172032 A
- US 2007214999 A1
- US 20070214999 A